# EUROPEAN PATENT APPLICATION

(11) **EP 2 586 779 A1**
(43) Date of publication of application: **01.05.2013**
(21) Application number: 12189770.6
(22) Date of filing: 24.10.2012
(51) Int. Cl.: C07D 403/12

(54) **Process for the preparation of moxonidine**

(30) Priority: 24.10.2011 IN CH36342011
(71) Applicant: Hetero Research Foundation, Hyderabad 500 018 (IN)
(72) Inventor: Parthasaradhi Reddy, Bandi, 500018 Andhra Pradesh (IN); Rathnakar Reddy, Kura, 500018 Andhra Pradesh (IN); Muralidhara Reddy, Dasari, 500018 Andhra Pradesh (IN); Ramakrishna Reddy, Matta, 500018 Andhra Pradesh (IN); Vamsi Krishna, Bandi, 500018 Andhra Pradesh (IN)
(74) Representative: Thomas, Simon

(57) **Abstract**

The present invention provides an improved process for the preparation of 4,6-dichloro-2-methyl-5-(1-acetyl-2-imidazolin-2-yl)aminopyrimidine. The present invention also provides an improved process for the preparation of moxonidine. The present invention further provides a process for the purification of moxonidine.

## Description

This application claims the benefit of Indian Patent Application No. 3634/CHE/2011, filed on October 24, 2011, which is incorporated herein by reference.

### Filed of the Invention

The present invention provides an improved process for the preparation of 4,6-dichloro-2-methyl-5-(1-acetyl-2-imidazolin-2-yl)aminopyrimidine. The present invention also provides an improved process for the preparation of moxonidine. The present invention further provides a process for the purification of moxonidine.

### Background of the Invention

Moxonidine is chemically, 4-chloro-N-(4,5-dihydro-1H-imidazol-2-yl)-6-methoxy-2-methyl-5-pyrimidinamine and has the structural formula:

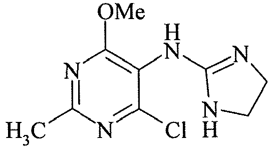

Moxonidine is a new generation centrally acting antihypertensive drug licensed for the treatment of mild to moderate essential hypertension. It may have a role when thiazides, beta-blockers, ACE inhibitors and calcium channel blockers are not appropriate or have failed to control blood pressure. In addition, it demonstrates favorable effects on parameters of the insulin resistance syndrome, apparently independent of blood pressure reduction. It is manufactured by Solvay Pharmaceuticals under the brand name Physiotens.

Moxonidine and its process were disclosed in U.S. patent no. 4,323,570 ('570 patent). According to the '570 patent, 4,6-dichloro-2-methyl-5-(1-acetyl-2-imidazolin-2-yl)-aminopyrimidine can be prepared by reacting 5-amino-4,6-dichloro-2-methylpyrimidine with 1-acetyl-2-imidazolidin-2-one in the presence of 10 volumes of phosphorous oxide trichloride at 50°C.

A process for preparing moxonidine was disclosed in European patent application publication no. 1982983. According to the publication, moxonidine can be prepared by reacting 4,6-dichloro-2-methyl-5-(1-acetyl-2-imidazolin-2-yl)amino-pyrimidine with sodium methoxide in an organic solvent and water and isolating.

European patent application publication no. 1894926 disclosed Form I, Form II and Form III of moxonidine.

4,6-Dichloro-2-methyl-5-(1-acetyl-2-imidazolin-2-yl)aminopyrimidine is a key intermediate for the preparation of moxonidine.

We have found an improved process for the preparation of 4,6-dichloro-2-methyl-5-(1-acetyl-2-imidazolin-2-yl)aminopyrimidine. The process of the invention results in higher yields compared with the known process of using more than 8 volumes of phosphorus oxide trichloride in the reaction.

We have also found an improved process for the preparation of moxonidine. The process of the invention results in higher yields and better impurity profile compared with the known process.

4,6-Dichloro-N-(imidazolidin-2-yldene)-2-methylpyrimidin-5-amine (6-chloromoxonidine) and N-(imidazolidin-2-ylidene)-4,6-dimethoxy-2-methylpyrimidin-5-amine (4-methoxymoxonidine) are potential impurities in moxonidine.

The chemical formula of 6-chloromoxonidine may be represented as:

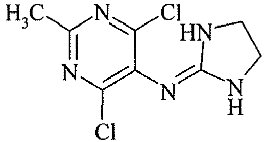

The chemical formula of 4-methoxymoxonidine may be represented as:

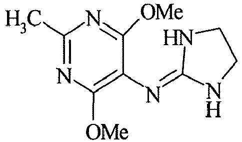

The present invention is intended to enhance the purity of moxonidine. In particular, the present invention is directed to reduce or remove 6-chloromoxonidine and 4-methoxymoxonidine impurities from moxonidine.

Thus, one object of the present invention is to provide an improved process for the preparation of 4,6-dichloro-2-methyl-5-(1-acetyl-2-imidazolin-2-yl)aminopyrimidine.

Another object of the present invention is to provide an improved process for the preparation of moxonidine.

Another object of the present invention is to provide a process for the purification of moxonidine.

### Summary of the Invention

In one aspect, the present invention provides an improved process for the preparation of 4,6-dichloro-2-methyl-5-(1-acetyl-2-imidazolin-2-yl)aminopyrimidine, which comprises reacting the 4,6-dichloro-2-methyl-5-pyrimidinamine with 1-acetyl-2-imidazolidine-2-one in the presence of phosphorous oxide trichloride **characterized in that** the quantity of phosphorous oxide trichloride is 2 to 4 volumes with respect to 4,6-dichloro-2-methyl-5-pyrimidinamine.

In another aspect, the present invention provides an improved process for the preparation of moxonidine, which comprises reacting the 4,6-dichloro-2-methyl-5-(1-acetyl-2-imidazolin-2-yl)aminopyrimidine with sodium methoxide in the presence of an alcoholic solvent at about 30 to 35°C.

Yet in another aspect, the present invention provides a process for the purification of moxonidine, which comprises:
a) suspending moxonidine in a mixture of an alcoholic solvent and an ester solvent;
b) heating the suspension obtained in step (a) at above 50°C;
c) cooling the reaction mass obtained in step (b) at below 20°C; and
d) isolating highly pure moxonidine.

### Detailed Description of the Invention

The term "room temperature" refers to temperature at about 20 to 30°C.

According to one aspect of the present invention, there is provided an improved process for the preparation of 4,6-dichloro-2-methyl-5-(1-acetyl-2-imidazolin-2-yl)aminopyrimidine, which comprises reacting the 4,6-dichloro-2-methyl-5-pyrimidinamine with 1-acetyl-2-imidazolidine-2-one in the presence of phosphorus oxychloride **characterized in that** the quantity of phosphorus oxychloride is 2 to 4 volumes with respect to 4,6-dichloro-2-methyl-5-pyrimidinamine.

The process may preferably be carried out in a solvent selected from methanol, ethanol, isopropanol, ethyl acetate, methyl acetate, isopropyl acetate, tert-butyl methyl acetate or a mixture thereof. More preferably the solvents are methanol and ethyl acetate.

Preferably the process may be carried out in the presence of a base selected from sodium hydroxide, potassium hydroxide, calcium hydroxide, sodium carbonate, potassium carbonate, calcium carbonate, sodium bicarbonate, potassium bicarbonate or calcium bicarbonate. More preferably the base is potassium carbonate.

According to another aspect of the present invention, there is provided an improved process for the preparation of moxonidine, which comprises reacting the 4,6-dichloro-2-methyl-5-(1-acetyl-2-imidazolin-2-yl)aminopyrimidine with sodium methoxide in the presence of an alcoholic solvent at about 30 to 35°C.

The alcoholic solvent used in the process may preferably be a solvent or a mixture of solvents selected from methanol, ethanol, isopropanol and n-butanol, and more preferably the alcoholic solvent is methanol.

According to another aspect of the present invention, there is provided a process for the purification of moxonidine, which comprises:
a) suspending moxonidine in a mixture of an alcoholic solvent and an ester solvent;
b) heating the suspension obtained in step (a) at above 50°C;
c) cooling the reaction mass obtained in step (b) at below 20°C; and
d) isolating highly pure moxonidine.

The term "highly pure moxonidine" refers to moxonidine having the purity greater than about 98% by weight, preferably greater than about 99% by weight, and more preferably greater than about 99.5% by weight.

The alcoholic solvent used in step (a) may preferably be a solvent or a mixture of solvents selected from methanol, ethanol, isopropanol and n-butanol, and more preferably the alcoholic solvent is methanol.

Preferably the ester solvent used in step (a) may be a solvent or a mixture of solvents selected from ethyl acetate, methyl acetate, isopropyl acetate, tert-butyl methyl acetate and ethyl formate. More preferably the ester solvent is ethyl acetate.

The reaction in step (b) may preferably be heated at about 55 to 75°C.

The reaction mass may preferably be cooled in step (c) at about 0 to 10°C and more preferably at about 0 to 5°C.

Highly pure moxonidine may be isolated in step (d) by methods known such as filtration or centrifugation.

The contents of moxonidine and the impurities are determined by High performance liquid chromatography (HPLC).

The invention will now be further described by the following example, which is illustrative rather than limiting.

### Examples

### Example 1:

### Preparation of 4,6-dichloro-2-methyl-5-(1-acetyl-2-imidazolin-2-yl)aminopyrimidine

Phosphorus oxychloride (1500 ml), 4,6-dichloro-2-methyl-5-pyrimidinamine (500 gm) and 1-acetyl-2-imidazolidine-2-one (400 gm) were added at room temperature. The contents were heated to 50°C, maintained for 48 hours and the phosphorus oxychloride was distilled under vacuum at below 45°C. The reaction mass was poured into chilled water at 5°C for 1 hour to 1 hour 30 minutes. The pH of the reaction mass was adjusted to 8.5 with potassium carbonate solution, stirred for 30 minutes and filtered. The solid thus obtained was added methanol (8750 ml) and ethyl acetate (8750 ml) at room temperature. The contents were heated to reflux and treated with carbon. The reaction mass was filtered through ciliate bed and then dried to obtain 560 gm of 4,6-dichloro-2-methyl-5-(1-acetyl-2-imidazolin-2-yl)aminopyrimidine.

### Example 2:

### Preparation of moxonidine

Methanol (18000 ml) was added to 4,6-dichloro-2-methyl-5-(1-acetyl-2-imidazolin-2-yl)aminopyrimidine (600 gm) at room temperature and then heated to 30 to 35°C. To the reaction mass was added sodium methoxide (600 gm) slowly for 45 minutes. The solvent was distilled under vacuum below 30°C to obtain a residual solid and then added water (5500 ml). The reaction mass was stirred for 10 minutes and filtered. The solid thus obtained was dried to get 520 gm of moxonidine.
Chromatographic purity of moxonidine: 99.78%;
Content of 6-chloromoxonidine: 0.03%;
Content of 4-methoxymoxonidine: 0.15%.

### Example 3:

### Purification of moxonidine

Moxonidine (520 gm, HPLC Purity: 99.78%) as obtained in example 2 was suspended in a mixture of methanol and ethyl acetate (17680 ml; 1:1) at room temperature. The reaction mixture was heated to 60°C and stirred for 45 minutes at same temperature. The reaction mass was filtered on paper and distilled under vacuum. The reaction mass was stirred for 45 minutes at 0 to 5°C and filtered. The solid obtained was dried to give 465 gm of pure moxonidine.
Chromatographic purity of moxonidine: 99.9%;
Content of 6-chloromoxonidine: 0.02%;
Content of 4-methoxymoxonidine: 0.05%.

### Example 4:

### Preparation of moxonidine Form I

Moxonidine (2 gm) was suspended in a mixture of acetonitrile and water (60 ml, 1:1) and then heated to 90°C to obtain a solution. The solution was then cooled to room temperature and stirred for 5 hours. The separated solid was filtered and then dried to obtain 1.8 gm of moxonidine Form I.

### Example 5:

### Preparation of moxonidine Form I

Moxonidine (2 gm) was suspended in dimethyl sulfoxide (12 ml) and then heated to 99°C to obtain a solution. To the solution was added a mixture of acetonitrile and water (24 ml, 1:1) at 0°C and stirred for 1 hour. The separated solid was filtered and then dried to obtain 1.6 gm of moxonidine Form I.

### Example 6:

### Preparation of moxonidine Form I

Moxonidine (2 gm) was suspended in a mixture of methanol and water (200 ml, 2:8) and then heated to 90°C to obtain a solution. The solution was then cooled to room temperature and stirred for 5 hours. The separated solid was filtered and then dried to obtain 1.7 gm of moxonidine Form I.

### Example 7:

### Preparation of moxonidine Form I

Moxonidine (2 gm) was suspended in dimethyl sulfoxide (12 ml) and then heated to 99°C to obtain a solution. To the solution was added acetone (24 ml) at -10°C and stirred for 1 hour. The separated solid was filtered and then dried to obtain 1.6 gm of moxonidine Form I.

### Example 8:

### Preparation of moxonidine Form I

Moxonidine (2 gm) was suspended in dimethyl sulfoxide (12 ml) and then heated to 99°C to obtain a solution. To the solution was added methanol (24 ml) at -10°C, stirred for 1 hour and filtered. The solid thus obtained was dried to obtain 1.5 gm of moxonidine Form I.

### Example 9:

### Preparation of moxonidine Form I

Moxonidine (2 gm) was suspended in dimethyl sulfoxide (12 ml) and then heated to 99°C to obtain a solution. To the solution was added water (24 ml) at 0°C and stirred for 1 hour. The separated solid was filtered and then dried to obtain 1.7 gm of moxonidine Form I.

## Claims

1. A process for the preparation of 4,6-dichloro-2-methyl-5-(1-acetyl-2-imidazolin-2-yl)aminopyrimidine, which comprises reacting 4,6-dichloro-2-methyl-5-pyrimidinamine with 1-acetyl-2-imidazolidine-2-one in the presence of phosphorus oxychloride **characterized in that** the quantity of phosphorus oxychloride is 2 to 4 volumes with respect to 4,6-dichloro-2-methyl-5-pyrimidinamine.

2. The process according to claim 1, wherein the process is carried out in a solvent selected from methanol, ethanol, isopropanol, ethyl acetate, methyl acetate, isopropyl acetate, tert-butyl methyl acetate or a mixture thereof.

3. The process according to claim 1 or claim 2, wherein the process is carried out in the presence of a base selected from sodium hydroxide, potassium hydroxide, calcium hydroxide, sodium carbonate, potassium carbonate, calcium carbonate, sodium bicarbonate, potassium bicarbonate or calcium bicarbonate.

4. A process for the preparation of moxonidine, which comprises reacting 4,6-dichloro-2-methyl-5-(1-acetyl-2-imidazolin-2-yl)aminopyrimidine with sodium methoxide in the presence of an alcoholic solvent at about 30 to 35°C.

5. The process according to claim 4, wherein the alcoholic solvent used in the process is a solvent or a mixture of solvents selected from methanol, ethanol, isopropanol and n-butanol.

6. A process for the purification of moxonidine, which comprises:
a. suspending moxonidine in a mixture of an alcoholic solvent and an ester solvent;
b. heating the suspension obtained in step (a) at above 50°C;
c. cooling the reaction mass obtained in step (b) at below 20°C; and
d. isolating highly pure moxonidine.

7. The process according to claim 6, wherein the alcoholic solvent used in step (a) is a solvent or a mixture of solvents selected from methanol, ethanol, isopropanol and n-butanol.

8. The process according to claim 6, wherein the ester solvent used in step (a) is a solvent or a mixture of solvents selected from ethyl acetate, methyl acetate, isopropyl acetate, tert-butyl methyl acetate and ethyl formate.

9. The process according to any one of claims 6 to 8, wherein the reaction in step (b) is heated at about 55 to 75°C.

10. The process according to any one of claims 6 to 9, wherein the reaction mass is cooled in step (c) at about 0 to 10°C.
